# EUROPÄISCHE PATENTSCHRIFT

(11) **EP 1 754 447 B1**
(45) Veröffentlichungstag und Bekanntmachung des Hinweises auf die Patenterteilung: **09.04.2008**
(21) Anmeldenummer: 06008508.1
(22) Anmeldetag: 25.04.2006
(51) Int. Cl.: A61B 17/16, A61C 1/00, H01H 13/00

(54) **Chirurgisches Elektrowerkzeug, Betätigungseinheit und Kalibrierungsverfahren hierfür**
Surgical electric tool, actuating unit and calibration method therefor
Outil électrique chirurgical, unité d'actionnement et procédé d'étalonnage correspondant

(30) Priorität: 17.08.2005 DE 102005038864
(43) Veröffentlichungstag der Anmeldung: 21.02.2007
(73) Patentinhaber: Stryker Leibinger GmbH & Co. KG, 79111 Freiburg (DE)
(72) Erfinder: Ippisch, Andreas, 79291 Merdingen (DE)
(74) Vertreter: Röthinger, Rainer

(56) Entgegenhaltungen:
- US-A- 6 037 724
- US-A1- 2002 049 464
- US-B1- 6 500 169

## Beschreibung

### Gebiet der Erfindung

Die Erfindung betrifft eine fingerkraftgesteuerte Betätigungseinheit für ein chirurgisches Elektrowerkzeug. Die Erfindung betrifft ferner ein chirurgisches Elektrowerkzeug mit einer derartigen Betätigungseinheit sowie ein Kalibrierungsverfahren für das Elektrowerkzeug.

### Hintergrund der Erfindung

Bereits seit mehreren Jahrzehnten unterstützen chirurgische Elektrowerkzeuge die Chirurgen bei ihren Tätigkeiten. Als Beispiele für chirurgische Elektrowerkzeuge können elektromotorisch betriebene chirurgische Bohrer, Knochensägen und Schraubendreher für Knochenschrauben angeführt werden.

Jedes chirurgische Elektrowerkzeug umfasst eine Betätigungseinheit wie beispielweise einen beweglichen Schalter oder einen Drehknopf zur Steuerung bestimmter Funktionalitäten des Werkzeugs. Im einfachsten Fall kann es sich bei der Betätigungseinheit um einen Kippschalter handeln, um das Elektrowerkzeug wahlweise ein- oder auszuschalten. Häufig werden mehrstufige Schalter verwendet, damit ein Benutzer des Elektrowerkzeugs zwischen verschiedenen Betriebsarten auswählen kann. Zur Einstellung einer Drehzahl des Elektrowerkzeugs gelangen auch drehbare Betätigungseinheiten wie stufenlose Drehknöpfe zum Einsatz.

Herkömmliche mechanische Betätigungseinheiten wie Kippschalter oder Drehknöpfe sind für chirurgische Elektrowerkzeuge, jedenfalls sofern diese sterilisiert werden müssen, häufig ungeeignet. Dies hängt damit zusammen, dass solche Betätigungseinheiten eine Vielzahl von beweglichen mechanischen Komponenten umfassen, die sich schlecht gegenüber dem Eintritt von flüssigen oder gasförmigen Sterilisationsmedien abdichten lassen. Das Eindringen eines Sterilisationsmediums in derartige Betätigungseinheiten ist deren Funktionsfähigkeit abträglich. Aus diesem Grund lassen sich chirurgische Elektrowerkzeuge mit Kippschaltern, Drehknöpfen oder ähnlichen mechanischen Betätigungseinheiten entweder überhaupt nicht sterilisieren oder müssen nach wenigen Sterilisationszyklen gewartet werden.

Um die Sterilisierbarkeit chirurgischer Elektrowerkzeuge zu verbessern oder überhaupt erst zu ermöglichen, werden bei solchen Werkzeugen die mechanischen Betätigungseinheiten häufig durch eine Kombination aus einem Kraftsensor und einer Signalaufbereitungsschaltung für den Kraftsensor ersetzt. Kraftsensoren weisen in der Regel eine flächige Gestalt auf und besitzen keine beweglichen mechanischen Elemente. Aus diesem Grund lassen sich Kraftsensoren auf einfache und dichte Weise unterhalb eines flexiblen Gehäuseabschnitts eines chirurgischen Elektrowerkzeugs einbauen.

Chirurgische Elektrowerkzeuge mit einem unterhalb eines flexiblen Gehäuseabschnitts angeordneten Kraftsensor sind beispielweise aus der US 3,463,990 oder der US 6,037,724 bekannt. Bei solchen chirurgischen Elektrowerkzeugen ist der Kraftsensor üblicherweise innerhalb einer Kunststoffummantelung aufgenommen, die den Kraftsensor (sowie häufig auch die zugehörige Signalaufbereitungsschaltung) gegenüber einem Sterilisationsmedium schützt. In der Praxis hat sich jedoch herausgestellt, dass trotz dieser Ummantelung bereits nach wenigen Sterilisationszyklen eine Wartung oder ein Austausch der sensorbasierten Betätigungseinheiten erforderlich ist.

Der Erfindung liegt die Aufgabe zugrunde, eine Betätigungseinheit für ein chirurgisches Elektrowerkzeug anzugeben, die einer hohen Anzahl von Sterilisationszyklen standhält. Der Erfindung liegt ferner die Aufgabe zugrunde, ein chirurgisches Elektrowerkzeug bereitzustellen, das besser sterilisierbar ist. Eine weitere, der Erfindung zugrunde liegende Aufgabe besteht darin, ein Kalibrierungsverfahren für das Elektrowerkzeug anzugeben.

### Kurzer Abriss der Erfindung

Gemäß einem ersten Aspekt der Erfindung wird eine Betätigungseinheit für ein chirurgisches Elektrowerkzeug bereitgestellt. Die Betätigungseinheit umfasst eine hermetisch abgedichtete metallische Kapsel zur Anordnung im Bereich eines Gehäuses des chirurgischen Werkzeugs, wobei die Kapsel auf einer Außenseite eine Auflagefläche für einen Finger definiert. Die Betätigungseinheit umfasst ferner einen innerhalb der Kapsel angeordneten Kraftsensor, der sich in kraftübertragender Verbindung mit der Auflagefläche befindet, und wenigstens einen, aus der Kapsel heraus führenden elektrischen Kontakt.

Die Kapsel kann in, auf oder unter dem Gehäuse des chirurgischen Elektrowerkzeugs angeordnet werden. Zweckmäßigerweise bestehen zumindest diejenigen Bereiche der Kapsel aus einem gegenüber Sterilisationsmedien beständigen Metall (oder sind mit einem solchen Metall beschichtet), welche in einem bezüglich des Sterilisationsmediums exponierten Gehäusebereich angeordnet sind. Die Kapsel kann aus einem Metall gefertigt sein und erforderlichenfalls einen Überzug aus Kunststoff oder aus einem anderen Material besitzen. Die Kapsel kann aber auch einen Kern aus einem nicht-metallischen Werkstoff besitzen, der mit einer Metallbeschichtung überzogen ist.

Um dem Chirurgen das Auffinden der Betätigungseinheit auch dann zu ermöglichen, wenn sein Blick von dem chirurgischen Elektrowerkzeug abgewandt ist, kann die Auflagefläche eine klar definierte räumliche Begrenzung besitzen. Die räumliche Begrenzung ist für den Chirurgen haptisch erfassbar und vereinfacht damit die Betätigung des chirurgischen Elektrowerkzeugs.

Was den Aufbau des Kraftsensors anbelangt, kann zwischen verschiedenen Realisieren ausgewählt werden. So ist es denkbar, den Kraftsensor als Dehnungsmessstreifen, als Piezoelement, als Halbleiterelement, usw. auszubilden. Der Kraftsensor kann auf einer der Auflagefläche gegenüberliegenden Innenfläche der Kapsel angeordnet sein. Wird die Auflagefläche von einer Oberseite einer Kapselwand gebildet, kann der Kraftsensor demgemäß unmittelbar auf einer der Auflagefläche gegenüberliegenden Unterseite der Kapselwand befestigt werden. Die Befestigung des Kraftsensors kann mittels Klebens oder auf irgendeine andere Weise erfolgen.

Für den Kraftsensor ist zweckmäßigerweise eine mit dem Kraftsensor gekoppelte Signalaufbereitungsschaltung vorgesehen. Die Signalaufbereitungsschaltung kann außerhalb oder innerhalb der Kapsel angeordnet werden. Gemäß einer ersten Variante greift die Signalaufbereitungsschaltung ein Sensorsignal ab und wandelt dieses in ein betätigungskraftabhängiges, kontinuierliches Ausgangssignal. Gemäß einer zweiten Variante wandelt die Signalaufbereitungsschaltung das Sensorsignal in ein diskretes (z. B. binäres oder mehrstufiges) Ausgangssignal.

Die Kapsel kann unterschiedliche Formen annehmen. So kann die Kapsel eine zylindrische und insbesondere eine kurzzylindrische (pillenförmige) Gestalt besitzen. Gemäß einer Ausführungsform weist die Kapsel eine topfförmige metallische Kappe und einen die Kappe abschließenden Kapselboden auf. Bei dieser Ausführungsform ist die Auflagefläche auf einer Oberseite der Kappe oder am Kapselboden ausgebildet. Der Kapselboden, welcher der Kappenoberseite gegenüberliegt, kann je nach Exponiertheit bezüglich des Sterilisationsmediums aus einem nicht-metallischen Werkstoff bestehen oder einen metallischen Werkstoff beinhalten.

Um einen Signalabgriff zu ermöglichen oder um Signale aus der Kapsel auszuleiten, kann die Kapsel eine oder mehrere Öffnungen besitzen, durch welche sich der wenigstens eine elektrische Kontakt hindurch erstreckt. Die Öffnungen sind vorzugsweise in einer Seitenwand oder auf einer Unterseite der Kapsel ausgebildet und hermetisch verschlossen. Zum Verschließen der Öffnungen ist Glas oder ein ähnlich beständiger Werkstoff geeignet.

Die Kapsel zur Aufnahme des Kraftsensors kann speziell gemäß den Erfordernissen des jeweiligen chirurgischen Elektrowerkzeugs gefertigt sein. Als kostengünstige Alternative hierzu kann es sich bei der Kapsel aber auch um ein Standardelement (wie beispielweise ein Standard-Transistorgehäuse) handeln.

Gemäß einem weiteren Aspekt der Erfindung wird ein chirurgisches Elektrowerkzeug bereitgestellt. Das chirurgische Elektrowerkzeug umfasst ein Gehäuse, einen Elektromotor sowie wenigstens eine fingerkraftgesteuerte und im Bereich des Gehäuses angeordnete Betätigungseinheit mit einem hermetisch abgedichteten Kraftsensor. Die Betätigungseinheit weist eine hermetisch abgedichtete metallische Kapsel zur Anordnung im Bereich des Gehäuses des chirurgischen Elektrowerkzeugs auf, wobei die Kapsel auf einer Aussenseite eine Auflagefläche für einen Finger definiert. Weiterhin weist sie einen innerhalb der Kapsel angeordneten Kraftsensor auf, sowie wenigstens einen aus der Kapsel heraus führenden elektrischen Kontakt. Der Kraftsensor befindet sich mit dieser Auflagefläche in kraftübertragender Verbindung.

Der Gehäuseeinsatz kann von einer hermetisch abgedichteten metallischen Kapsel gebildet sein, innerhalb welcher der Kraftsensor angeordnet ist. Die Auflagefläche zur Krafteinleitung kann in diesem Fall in Form einer Außenseite der Kapsel vorgesehen werden. Der metallische Gehäuseeinsatz muss jedoch nicht notwendigerweise eine Kapselungsfunktion für den Kraftsensor besitzen. Vielmehr könnte der metallische Gehäuseeinsatz auch eine im Wesentlichen planare, erforderlichenfalls an eine Wölbung des Gehäuses angepasste Gestalt besitzen, wobei dann besondere Vorkehrungen zum hermetischen Abdichten des Kraftsensors erforderlich werden können. Solche Vorkehrungen können eine hermetisch dichte Verbindung des metallischen Gehäuseeinsatzes mit den sich an den Gehäuseeinsatz anschließenden Gehäusebereichen beinhalten. Der metallische Gehäuseeinsatz kann aus einem (z.B. zwei- oder mehrschichtigen) Verbundmaterial bestehen. Dabei umfasst der Gehäuseeinsatz mindestens eine metallische Schicht.

Der metallische Bereich des Gehäuseeinsatzes, welcher die Fingerkraft auf den Kraftsensor überträgt, besitzt zweckmäßigerweise bezüglich der einzuleitenden Fingerkraft elastische (federnde) Eigenschaften. Um elastische Eigenschaften zu erzielen, können Fläche und Stärke des metallischen Bereiches geeignet gewählt sein. Bei zu hoher Materialstärke wird die Fingerkraft, welche zur Erzielung einer vom Kraftsensor erfassbaren Deformation aufzubringen ist, zu hoch. Andererseits besteht bei einer zu niedrigen Materialstärke die Gefahr, dass die Fingerkraft zu einer nicht reversiblen (plastischen) Verformung führt. In Abhängigkeit vom gewählten metallischen Material wird der Fachmann unter Berücksichtigung der Größe der Auflageflächen in der Lage sein, eine zwischen diesen beiden Grenzbereichen liegende, geeignete Materialstärke zu wählen. Bei handelsüblichen sterilisierbaren metallischen Materialien (wie z.B. NiCo-Stählen) betragen typische Materialstärken bei fingertypischen Auflageflächengrößen ungefähr 0,05 mm bis 1,0 mm, zweckmäßigerweise ungefähr 0,1 bis 0,4 mm.

Zur Verbesserung der Handhabung des chirurgischen Elektrowerkzeuges kann die Auflagefläche über eine Oberfläche des Gehäuses überstehen oder bezüglich der Oberfläche versenkt angeordnet sein. Eine solche Maßnahme erleichtert dem Chirurgen das haptische Auffinden der Auflagefläche und damit die Werkzeugbedienung. Zusätzlich oder alternativ hierzu kann die Auflagefläche eine Oberflächenstrukturierung besitzen (z.B. nach Art einer Riffelung).

Ein einzelnes Elektrowerkzeug kann ein, zwei oder mehr Betätigungseinheiten umfassen. So ist es denkbar, dass eine erste Betätigungseinheit zur Steuerung des Elektromotors in einer ersten Drehrichtung und eine zweite Betätigungseinheit zur Steuerung des Elektromotors in einer zur ersten Drehrichtung entgegengesetzten zweiten Drehrichtung vorgesehen sind.

Für den Elektromotor kann eine Motoransteuerschaltung und für den Kraftsensor kann eine Signalaufbereitungsschaltung vorgesehen sein. Die Signalaufbereitungsschaltung umfasst zweckmäßigerweise eine Brückenschaltung, die auch den Kraftsensor beinhaltet.

Bei dem chirurgischen Elektrowerkzeug kann es sich um einen chirurgischen Bohrer, eine chirurgische Säge oder einen Schraubendreher (z. B. für Knochenschrauben) handeln. Gemäß einer ersten Variante besitzt das Elektrowerkzeug ein pistolenförmiges Gehäuse. Gemäß einer zweiten Variante weist das Elektrowerkzeug ein langgestrecktes und zumindest annähernd zylindrisches Gehäuse auf, wobei die wenigstens eine Betätigungseinheit in einem vorderen (also dem eigentlichen Werkzeug wie einem Bohrer oder einer Schraubendreherklinge zugewandten) Gehäuseabschnitt angeordnet ist.

Gemäß einem dritten Aspekt wird die Verwendung eines metallischen Standard-Transistorgehäuses (oder von Teilen hiervon) zur hermetischen Verkapselung eines Kraftsensors für eine Betätigungseinheit eines chirurgischen Elektrowerkzeuges vorgeschlagen. Bei dem Standard-Transistorgehäuse kann es sich um ein TO8- oder ein beliebiges anderes Transistorgehäuse handeln.

Gemäß einem weiteren Aspekt der Erfindung wird ein Verfahren zur Kalibrierung eines Kraftsensors, wie er in einer erfindungsgemäßen Betätigungseinheit oder bei einem erfindungsgemäßen chirurgischen Elektrowerkzeug zum Einsatz gelangen kann, bereitgestellt. Das Verfahren umfasst die Schritte des Erfassens eines vorbestimmten Ereignisses, das Messen eines Ausgangssignals des Kraftsensors als Antwort auf das Erfassen des vorbestimmten Ereignisses und das Verwenden des gemessenen Ausgangssignals als Nullsignal für den nächsten Betätigungsvorgang. Das Verfahren wird vorzugsweise im unbetätigten Zustand (ohne Fingerkraftbeaufschlagung) durchgeführt.

Bei dem vorbestimmten Ereignis kann es sich um das Aktivieren einer Spannungsversorgung einer Signalaufbereitungsschaltung oder um das Aktivieren des chirurgischen Elektrowerkzeugs selbst handeln. Beispielsweise können beim Einschalten des Elektrowerkzeugs oder beim Aufstecken eines Batteriepacks automatisch die oben genannten Schritte durchgeführt werden. Gemäß einer alternativen oder zusätzlichen Ausgestaltung beinhaltet das vorbestimmte Ereignis den Ablauf eines vorbestimmten Zeitintervalls, ohne dass ein Betätigungsvorgang stattgefunden hätte.

### Kurze Beschreibung der Zeichnungen

Weitere Aspekte und Vorteile der Erfindung ergeben sich aus der nachfolgenden Beschreibung bevorzugter Ausführungsbeispiele sowie aus dem Figuren. Es zeigt:
Fig. 1 eine schematische Teilschnittansicht durch ein erstes Ausführungsbeispiel einer Betätigungseinheit;
Fig. 2 eine Schnittansicht einer Kappe der Betätigungseinheit gemäß Fig. 1;
Fig. 3 ein Bodenteil für die Kappe gemäß Fig. 2 mit Kontakten in einer teilweisen Schnittdarstellung;
Fig. 4 eine Aufsicht auf eine Unterseite des Bodenteils gemäß Fig. 3 mit Kontaktbeschriftung;
Fig. 5 eine schematische Darstellung einer Signalaufbereitungsschaltung für die Betätigungseinheit;
Fig. 6 eine Seitenansicht eines ersten Ausführungsbeispiels eines chirurgischen Elektrowerkzeugs;
Fig. 7 eine Aufsicht auf das chirurgische Elektrowerkzeug gemäß Fi. 6;
Fig. 8 eine Schnittansicht des chirurgischen Elektrowerkzeugs gemäß Fig. 6;
Fig. 9 eine Logikschaltung zur Verwendung in dem chirurgischen Elektrowerkzeug gemäß den Fign. 6 bis 7;
Fig. 10 eine schematische Teilschnittansicht durch ein zweites Ausführungsbeispiel einer Betätigungseinheit;
Fig. 11 eine Schnittansicht einer Kappe der Betätigungseinheit gemäß Fig. 10;
Fig. 12 eine Aufsicht auf einen bei der Betätigungseinheit gemäß Fig. 10 zum Einsatz kommenden Dehnungsmessstreifen;
Fig. 13 eine ausschnittsweise Schnittansicht eines zweiten Ausführungsbeispiels eines chirurgischen Elektrowerkzeugs; und
Fig. 14 ein schematisches Flussdiagramm eines Ausführungsbeispiels eines Kalibrierungsverfahrens.

### Beschreibung bevorzugter Ausführungsbeispiele

Im Folgenden wird die Erfindung anhand bevorzugter Ausführungsbeispiele chirurgischer Elektrowerkzeuge und einer hierfür vorgesehenen Betätigungseinheit erläutert. Übereinstimmende Elemente sind mit übereinstimmenden Bezugszeichen versehen.

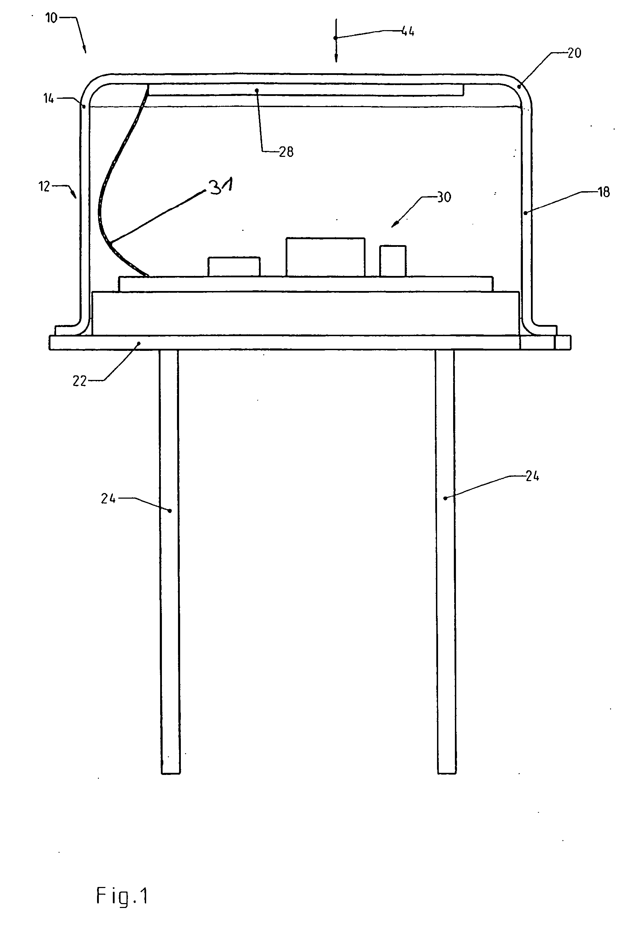

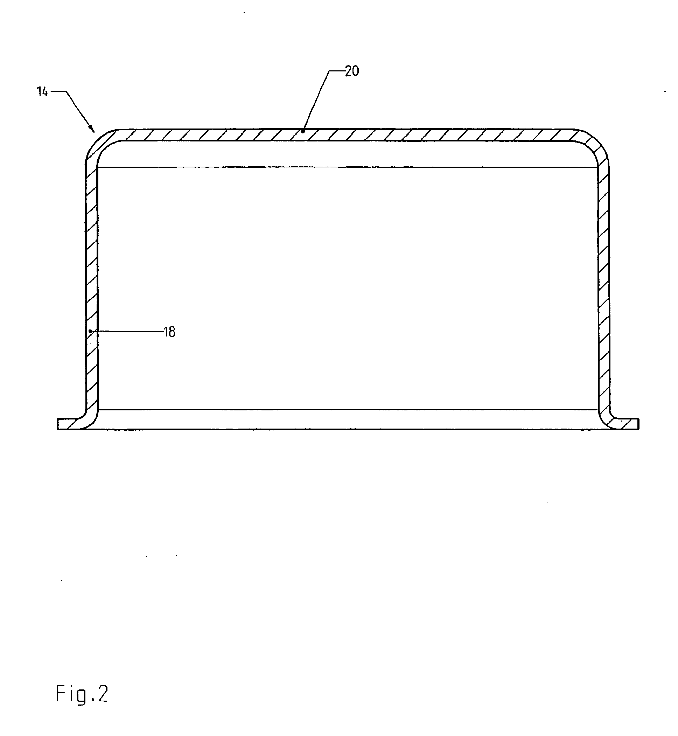

In Figur 1 ist eine Schnittansicht eines ersten Ausführungsbeispiels einer Betätigungseinheit 10 für ein chirurgisches Elektrowerkzeug dargestellt. Die Betätigungseinheit 10 umfasst eine hermetisch abgedichtete Kapsel 12 in Gestalt eines TO8-Transistor-gehäuses. Die Kapsel 12 besitzt eine im Wesentlichen topfförmige Kappe 14 aus Edelstahl, die in Figur 2 in einer stark vergrößerten Schnittansicht dargestellt ist. Die Kappe 14 umfasst einen zylindrischen Mantelabschnitt 18 sowie einen einstückig mit dem Mantelabschnitt 18 ausgebildeten Deckelabschnitt 20. Der Innendurchmesser des Mantelabschnitts 18 beträgt ungefähr 11 mm (typischerweise ca. 5 bis 30 mm) und die Höhe des Mantelabschnitts 18 ungefähr 7 mm (typischerweise ca. 2 bis 12 mm). Der Deckelabschnitt 20 verschließt die in den Figuren obere Stirnseite des Mantelabschnitts 18 und besitzt eine Materialstärke von ungefähr 0,25 mm. Die offene Stirnseite der Kappe 14 ist durch einen Kappenboden 22 hermetisch verschlossen. Der Kappenboden 22 besteht aus einem NiCo-Stahl.

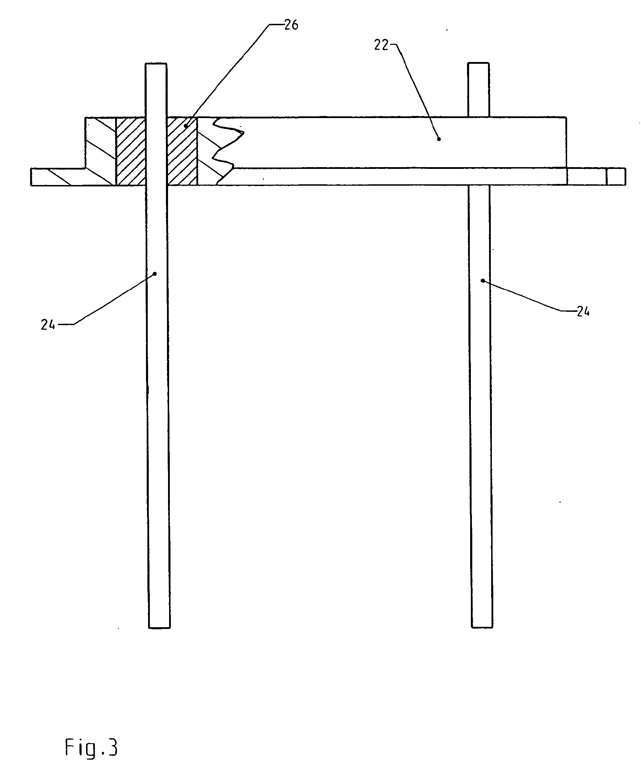

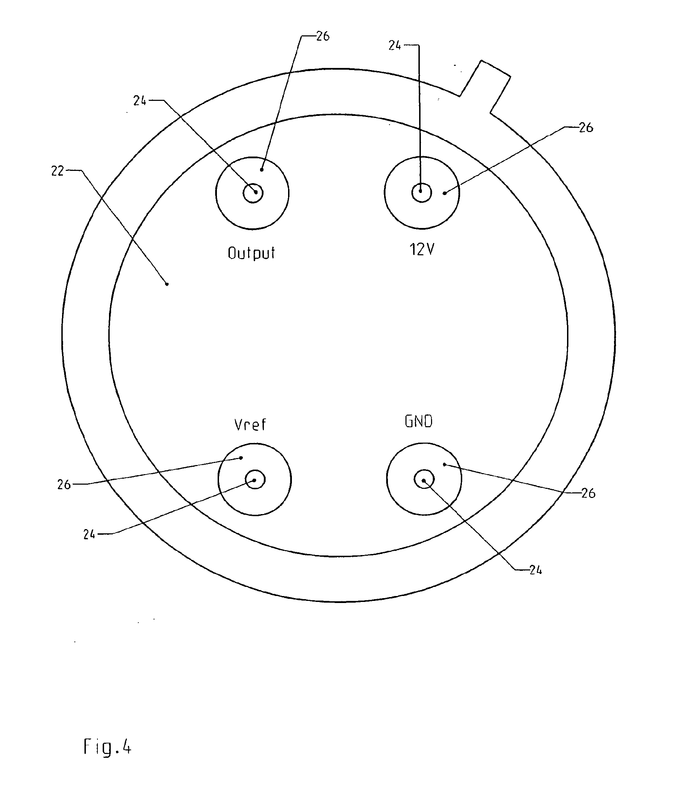

Wie sich aus den Figuren 3 und 4 ergibt, sind im Kappenboden 22 insgesamt vier Durchgangsöffnungen 26 ausgebildet. Durch jede Durchgangsöffnung 26 erstreckt sich ein vergoldeter elektrischer Kontakt 24. Um einerseits die Kontakte 24 zu stabilisieren und um andererseits eine hohe Dichtheit zu gewährleisten, sind die Öffnungen 26 mittels Glas hermetisch verschlossen.

Innerhalb der Kapsel 12 sind ein Kraftsensor in Gestalt eines Dehnungsmessstreifens 28 sowie eine Signalaufbereitungsschaltung 30 für den Kraftsensor 28 aufgenommen. Dieser Sachverhalt kann Figur 1 entnommen werden. Der Kraftsensor 28 ist mittels einer elektrischen Kontaktierung 31 mit der Signalaufbereitungsschaltung 30 gekoppelt. Die Signalaufbereitungsschaltung 30 wiederum wird von den aus der Kapsel 12 herausführenden Kontakten 24 elektrisch kontaktiert.

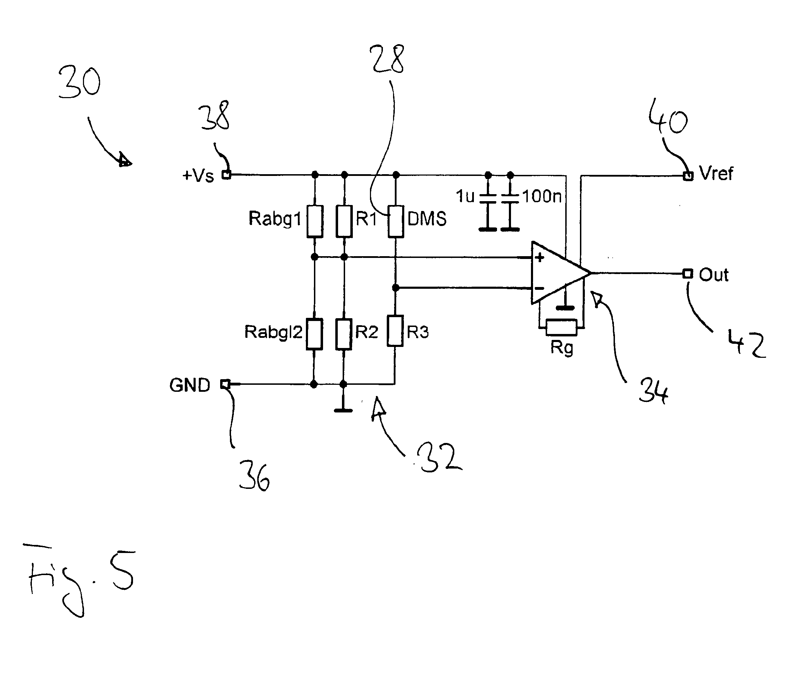

Figur 5 zeigt ein Schaltbild der Signalaufbereitungsschaltung 30. Wesentliche Bestandteile der Signalaufbereitungsschaltung 30 sind eine temperaturkompensierende Widerstands-Brückenschaltung 32 sowie eine Verstärkerkomponente 34. Der Kraftsensor 28 ist Teil der Brückenschaltung 32, die neben drei weiteren Brückenwiderständen R1, R2 und R3 auch noch zwei Abgleichwiderstände Rabg1, Rabg2 umfasst. In Übereinstimmung mit den vier aus der Kapsel 12 herausführenden Kontakten 24 (vgl. Fig. 4) umfasst die Signalaufbereitungsschaltung 30 vier Anschlüsse, nämlich einen Masseanschluss 36, den Anschluss 38 für eine Versorgungsspannung, einen Anschluss 40 für eine Referenzspannung sowie einen Anschluss 42 für ein Ausgangssignal der Verstärkerkomponente 34.

Die Funktionsweise der Betätigungseinheit 10 wird jetzt unter Bezugaufnahme auf die Figuren 1 und 5 ausführlicher erläutert. Die Oberseite des Kappendeckels 20 fungiert als Auflagefläche für einen Finger und gestattet das Einleiten einer Betätigungskraft entlang des Pfeils 44 in Figur 1. Wird auf den Kappendeckel 20 eine Betätigungskraft entlang des Pfeils 44 ausgeübt, verformt sich der Kappendeckel 20 elastisch in Richtung auf das Innere der Kapsel 12. Diese Verformung des Kappendeckels 20 überträgt sich auf den Kraftsensor 28, der beispielsweise mittels Klebens auf der Unterseite des Kappendeckels 20 befestigt ist. Genauer gesagt bewirkt die Verformung eine Dehnung des als Dehnungsmessstreifen ausgebildeten Kraftsensors 28. Infolge dieser Dehnung ändert sich der Widerstand des Kraftsensors 28. Diese Widerstandsänderung des Kraftsensors 28 wiederum verschiebt den Betriebspunkt der Brückenschaltung 32.

Die Verschiebung des Betriebspunktes wird von der als Differenzverstärker ausgebildeten Verstärkerkomponente 34 erfasst und in ein verstärktes Differenzsignal umgesetzt. Das verstärkte Differenzsignal wird als Ausgangssignal der Signalaufbereitungsschaltung 30 am Anschluss 42 zur Weiterverarbeitung bereitgestellt. Der Pegel des Ausgangssignals ist proportional zur Verformung des Dehnungsmessstreifens Kraftsensors 28 und damit auch proportional zu der in dem Kappendeckel 20 eingeleiteten Betätigungskraft. Bei einer alternativen Ausführungsform ist die Signalaufbereitungsschaltung derart ausgebildet, dass das Ausgangssignal zwei oder mehr diskrete Pegel (z. B. in Abhängigkeit von der Überschreitung einer oder mehrerer Kraftschwellen) besitzt.

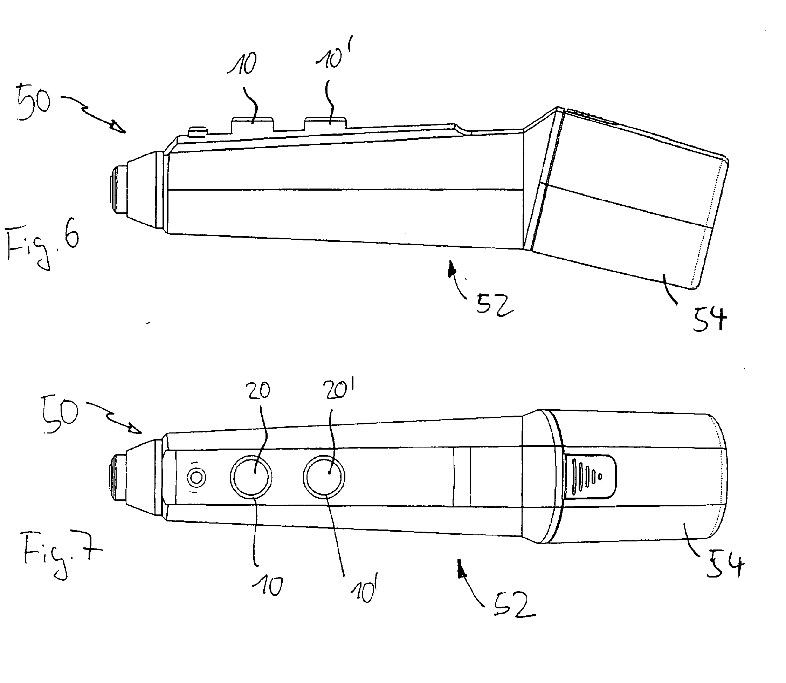

Jetzt wird unter Bezugnahme auf die Figuren 6 bis 8 ein erstes Ausführungsbeispiel eines chirurgischen Elektrowerkzeuges 50 in Gestalt eines batteriebetriebenen Schraubendrehers erläutert. Das chirurgische Elektrowerkzeug 50 besitzt ein langgestrecktes, annähernd zylindrisches Gehäuse 52, an das ein Batteriepack 54 abnehmbar aufgesteckt werden kann.

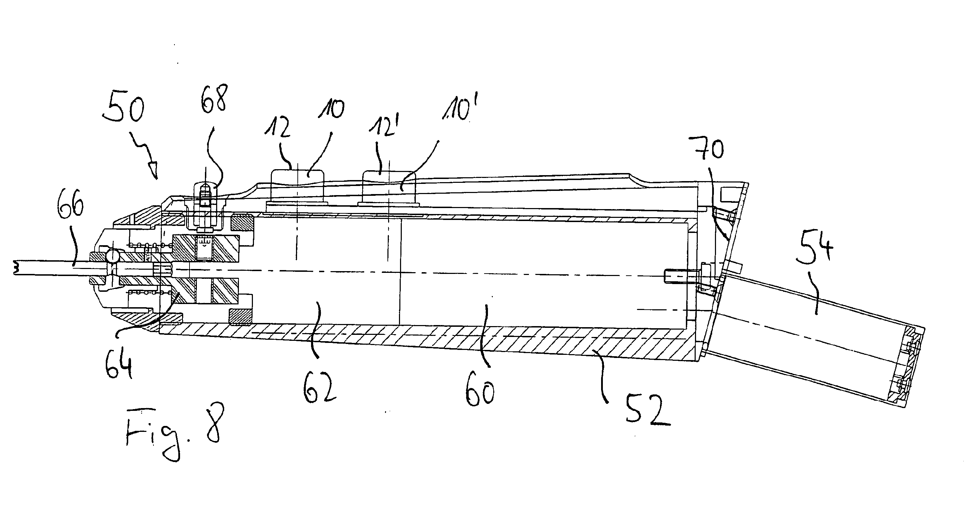

Das chirurgische Elektrowerkzeug 50 umfasst insgesamt zwei Betätigungseinheiten 10, 10'. Die Betätigungseinheiten 10, 10' sind in einem dem Batteriepack 54 abgewandten vorderen Bereich des Gehäuses 52 ausgebildet und besitzen den unter Bezugnahme auf die Figuren 1 bis 5 erläuterten Aufbau. Wie insbesondere der Figur 8 entnommen werden kann, erstrecken sich die Kappen der Betätigungseinheiten 10, 10' durch hierfür vorgesehene Öffnungen im Gehäuse 52 hindurch, so dass der jeweilige Kappendeckel 20, 20' etwas über das Gehäuse 52 übersteht. Es wäre jedoch auch denkbar, dass die Oberseiten der Betätigungseinheiten 10, 10' in einer Ebene mit der Oberfläche des Gehäuses 52 verlaufen oder versenkt bezüglich dieser Oberfläche angeordnet sind.

Wie in Figur 8 dargestellt, sind im Inneren des Gehäuses 52 ein elektronisch kommutierter Motor 60, ein mit dem Motor gekoppeltes Getriebe 62 sowie getriebeabwärts eine Kupplung 64 aufgenommen. Die Kupplung 64 gestattet in bekannter Weise die drehfeste Kopplung einer austauschbaren Schraubendreherklinge 66 mit dem Getriebe 62. Ein sich ebenfalls durch das Gehäuse 52 hindurch erstreckender Sperrknopf 68 ermöglicht eine drehfeste mechanische Arretierung der Kupplung 64. Ist der Sperrknopf 68 betätigt, lässt sich das Elektrowerkzeug 50 wie ein herkömmlicher Schraubendreher verwenden (das Drehmoment wird dann also nicht mittels des Motors 60 erzeugt, sondern durch manuelle Drehung des Gehäuses 52 aufgebracht).

Wie bereits erläutert, umfasst das chirurgische Elektrowerkzeug 50 insgesamt zwei Betätigungseinheiten 10, 10'. Eine erste der beiden Betätigungseinheiten 10, 10' steuert den Elektromotor 60 in einer ersten Drehrichtung ("Vorwärtsrichtung"). Die andere Betätigungseinheit steuert den Elektromotor 60 in einer zur ersten Drehrichtung entgegengesetzten zweiten Drehrichtung ("Rückwärtsrichtung"). Die Motordrehzahl in Vorwärts- und Rückwärtsrichtung ist jeweils proportional zur Betätigungskraft, die in die jeweilige Betätigungseinheit 10, 10' eingeleitet wird. Je höher die Betätigungskraft ist, desto höher ist die Motordrehzahl. Zur Drehzahlregelung ist eine Motoransteuerschaltung auf einer im rückwärtigen Teil des Gehäuses 52 befestigten Platine 70 untergebracht.

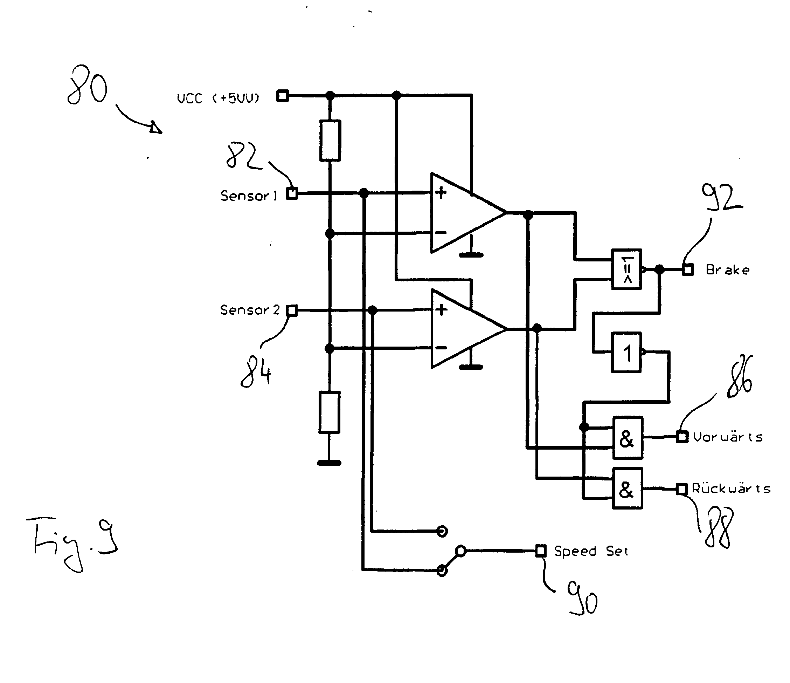

Die Motoransteuerungsschaltung ist elektrisch mit den Signalaufbereitungsschaltungen der Betätigungseinheiten 10, 10' gekoppelt. Funktionell zwischen der Motoransteuerungsschaltung und den beiden Betätigungseinheiten 10, 10' ist die in Figur 9 dargestellte Logikschaltung 80 angeordnet. Die Logikschaltung 80 bewirkt im Wesentlichen, dass sich bei gleichzeitiger Krafteinleitung in die beiden Betätigungseinheiten 10, 10' kein undefinierter Zustand einstellt. Zu diesem Zweck besitzt die Logikschaltung zwei getrennte Eingangsanschlüsse 82, 84, die jeweils mit einer der beiden Betätigungseinheiten 10, 10' gekoppelt sind. Sofern nur an einem einzigen der beiden Eingangsanschlüsse 82, 84 ein Signal anliegt, wird über genau einen von zwei Ausgangsanschlüssen 86, 88 ein verstärktes Ausgangssignal an die Motoransteuerschaltung weitergeleitet. Über den Anschluss 86 wird der Motoransteuerschaltung ein Signal für die erste Drehrichtung und über den Anschluss 88 ein Signal für die entgegengesetzte zweite Drehrichtung zugeführt.

Liegen an beiden Eingangsanschlüssen 82, 84 der Logikschaltung 80 Ausgangssignale an (d. h. wird in beide Betätigungseinheiten 10, 10' eine Betätigungskraft eingeleitet), bewirkt die in der Logikschaltung 10 implementierte Logik, dass an keinem der beiden Ausgangsanschlüsse 86, 88 ein Ausgangssignal an die Motoransteuerungsschaltung ausgegeben wird. Außerdem nimmt der "Brake"-Anschluss 92 einen hohen Signalpegel an. Der hohe Signalpegel am Anschluss 92 schließt den elektronisch kommutierten Elektromotor 60 kurz, wodurch der Elektromotor 60 elektrisch abgebremst wird und zum Stillstand gelangt.

Die in Fig. 9 dargestellte Logikschaltung 80 umfasst ferner einen Geschwindigkeitsregel-Anschluss 90. Über den Geschwindigkeitsregel-Anschluss 90 erhält die Motoransteuerungsschaltung eine Rückmeldung über die angeforderte Motordrehzahl.

Das unter Bezugnahme auf die Figuren 6 bis 9 beschriebene chirurgische Elektrowerkzeug 50 hält einer äußerst hohen Anzahl von Sterilisationszyklen innerhalb eines Autoklaven stand. Ein Grund hierfür sind die für ein Sterilisationsmedium undurchlässigen Betätigungseinheiten 10, 10' mit den in je einer Metallhülle verkapselten Kraftsensoren. Vorteilhaft ist ferner, dass trotz hoher Beständigkeit gegenüber einem Sterilisationsmedium die Betätigungseinheiten 10, 10' nicht notwendigerweise in das Gehäuse 52 flächig integriert zu werden brauchen, sondern einen Überstand über das Gehäuse 52 aufweisen können. Dies erleichtert die Handhabung des chirurgischen Elektrowerkzeuges 52 nachhaltig, da der Chirurg die Betätigungseinheiten 10, 10' problemlos "erfühlen" kann. Das chirurgische Elektrowerkzeug 52 kann daher auch ohne Sichtkontakt betätigt werden.

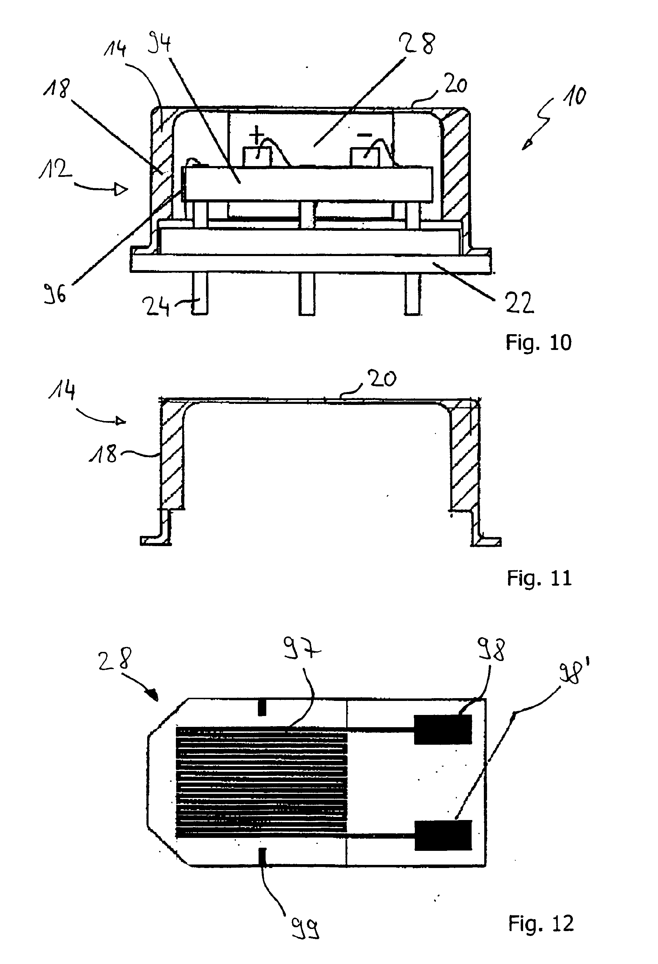

Nun werden unter Bezugnahme auf die Fign. 10 bis 13 weitere Ausführungsbeispiele einer erfindungsgemäßen Betätigungseinheit 10 sowie eines erfindungsgemäßen Elektrowerkzeugs 50 erörtert. Die Ausführungsbeispiele der Fign. 10 bis 13 stimmen in wesentlichen Aspekten mit den oben erläuterten Ausführungsbeispielen überein.

Aus diesem Grund sind korrespondierende Elemente mit übereinstimmenden Bezugszeichen versehen.

Fig. 10 zeigt eine Schnittansicht eines zweiten Ausführungsbeispiels einer gekapselten Betätigungseinheit 10 für ein chirurgisches Elektrowerkzeug. Innerhalb der Kapsel 12 ist eine Platine 94 einer Signalaufbereitungsschaltung aufgenommen. Die Signalaufbereitungsschaltung ist über eine Masseverbindung 96 mit dem Kappenboden 22 elektrisch verbunden. Während es sich jedenfalls bei der Kappe 14 der Kapsel 12 um eine Sonderanfertigung handelt, kann der Boden 22 von einem TO8-Transistorgehäuse stammen.

Die Schnittansicht gemäß Fig. 11 der aus Edelstahl gefertigten Kappe 14 zeigt die Unterschiede gegenüber dem ersten Ausführungsbeispiel. Ein maßgeblicher Unterschied besteht darin, dass der zylindrische Mantelabschnitt 18 eine deutlich größere Materialstärke als der Deckelabschnitt 20 besitzt. Während der Deckelabschnitt eine Stärke von ungefähr 0,3 mm aufweist, besitzt der Mantelabschnitt 18 eine Stärke von wenigstens ungefähr 0,8 mm oder darüber. Eine solche Ausführung ist vorteilhaft, um bei Einleiten einer Fingerkraft auf den Deckelabschnitt 20 die resultierenden elastischen Verformungen auf den Deckelabschnitt 20 zu beschränken. Mit anderen Worten verhält sich der Mantelabschnitt 18 im Wesentlichen starr bezüglich der in den Deckelabschnitt 20 eingeleiteten Betätigungskräfte. Dies erleichtert den hermetisch dichten Einbau der Betätigungseinheit 10 in das Gehäuse eines Elektrowerkzeugs.

Fig. 12 zeigt eine Aufsicht auf den in der Kapsel 12 gemäß Fig. 10 aufgenommenen Kraftsensor 28. Der Kraftsensor 28 umfasst eine Mäander-Struktur 97 eines Dehnungsmessstreifens mit zwei Kontaktierungen 98, 98'. Im fertig montierten Zustand sind die Kontaktierungen 98, 98' elektrisch mit der Signalaufbereitungsschaltung verbunden. Zwei Markierungen 99 deuten die Mitte der Mäander-Struktur 97 an und gestatten so eine zentrierte Montage innerhalb der Kapsel 12. Die Montage kann durch Verkleben erfolgen.

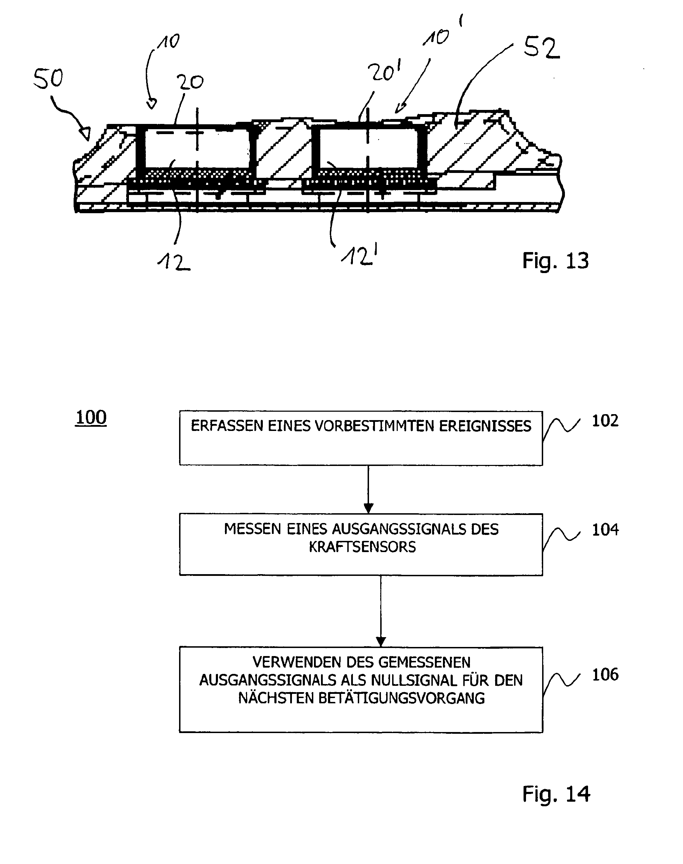

Fig. 13 zeigt eine Teilschnittansicht eines chirurgischen Elektrowerkzeugs 50 gemäß einem zweiten Ausführungsbeispiel. Deutlich zu erkennen sind die beiden Betätigungseinheiten 10, 10', welche gemäß dem in den Fign. 10 bis 12 erläuterten Ausführungsbeispiel ausgebildet sind. Gemäß Fig. 13 schließen die oberen Deckelabschnitte 20, 20' (also die Auflageflächen) bündig mit einer Oberseite des Gehäuses 52 des Elektrowerkzeugs 50 ab.

Im Folgenden wird unter Bezugnahme auf das Flussdiagramm 100 gemäß Figur 14 ein Ausführungsbeispiel eines Kalibrierungsverfahrens für die Kraftsensoren 28 des chirurgischen Elektrowerkzeugs 50 gemäß den Figuren 6 bis 9 und 13 erläutert. Dieses Kalibrierungsverfahren kann jedoch auch ganz allgemein bei Elektrowerkzeugen zum Einsatz gelangen, welche eine Betätigungseinheit 10 gemäß den Figuren 1 bis 5 oder 10 bis 12 beinhalten. Das Verfahren ist zweckmäßigerweise in Gestalt eines Software-Programms in dem entsprechenden Elektrowerkzeug hinterlegt.

Wie in Fig. 14 dargestellt, beginnt das Verfahren in einem ersten Schritt 102 damit, dass ein vorbestimmtes Ereignis erfasst wird. Bei diesem vorbestimmten Ereignis kann es sich um das Aufstecken des Batteriepacks 54 auf das chirurgische Elektrowerkzeug 50 und die damit einhergehende Aktivierung der Spannungsversorgung handeln. Zusätzlich oder alternativ hierzu kann es sich bei dem vorbestimmten Ereignis auch um den Ablauf eines vorbestimmten Zeitintervalls (von typischerweise 10 Sekunden bis 60 Sekunden, also z.B. von 30 Sekunden) handeln, ohne dass eine der Betätigungseinheiten 10, 10' aktiviert worden wäre.

In einem nächsten Schritt 104 wird für eine kurze Zeitspanne von typischerweise 0,5 Sekunden bis 5 Sekunden (z.B. für ca. 1 Stunde) das Ausgangssignal eines jeden Kraftsensors 28 gemessen.

In einem weiteren Schritt 106 wird das gemessene Ausgangssignal als Nullsignal für den folgenden Betätigungsvorgang gesetzt. Das Nullsignal, welches einer NichtBetätigung der Betätigungseinheiten 10, 10' entspricht, wird somit fortlaufend aktualisiert.

Eine solche fortlaufende Aktualisierung des Nullsignals hat verschiedene Vorteile, insbesondere im Zusammenhang mit den hier erläuterten Betätigungseinheiten. So werden sich dauerhafte (plastische) mechanische Verformungen der metallischen Kapsel 12, wie sie bei Schlägen oder beim Fallenlassen des Elektrowerkzeugs 50 entstehen können, automatisch kompensiert. Des Weiteren lässt sich Materialermüdungen sowie Deformationen zuverlässig entgegenwirken. Das chirurgische Elektrowerkzeug 50 bleibt damit fortlaufend betätigbar und regelbar. Außerdem ist das Elektrowerkzeug 50 wartungsfrei, da die Betätigungseinheiten 10, 10' keine beweglichen Teile umfassen und keine Nachjustage erfordern. Die damit einhergehenden Vorteile sind vor allem im chirurgischen Umfeld beträchtlich.

Selbstverständlich ist der Einsatzbereich der erfindungsgemäßen Betätigungseinheit nicht auf ein chirurgisches Elektrowerkzeug in Gestalt eines Schraubendrehers beschränkt. Vielmehr kann eine erfindungsgemäße Betätigungseinheit auch in anderen chirurgischen Elektrowerkzeugen wie Bohrern, Sägen, usw. Verwendung finden.

Es sind daher zahlreiche Modifikationen und Ergänzungen in Bezug auf die erfindungsgemäße Betätigungseinheit und das erfindungsgemäße chirurgische Elektrowerkzeug möglich. Der Umfang der Erfindung ist ausschließlich durch den Schutzbereich der nachfolgenden Ansprüche beschränkt.

## Patentansprüche

1. Betätigungseinheit (10) für ein chirurgisches Elektrowerkzeug (50), umfassend
- eine hermetisch abgedichtete metallische Kapsel (12) zur Anordnung im Bereich eines Gehäuses (52) des chirurgischen Elektrowerkzeugs (50), wobei die Kapsel (12) auf einer Außenseite eine Auflagefläche (20) für einen Finger definiert;
- einen innerhalb der Kapsel (12) angeordneten Kraftsensor (28), der sich in kraftübertragender Verbindung mit der Auflagefläche (20) befindet; und
- wenigstens einen, aus der Kapsel (12) heraus führenden elektrischen Kontakt (24).

2. Betätigungseinheit nach Anspruch 1, **dadurch gekennzeichnet,**
**dass** die Auflagefläche (20) eine räumliche Begrenzung besitzt, die haptisch erfassbar ist.

3. Betätigungseinheit nach Anspruch 1 oder 2, **dadurch gekennzeichnet,**
**dass** der Kraftsensor (28) einen Dehnungsmessstreifen (97) beinhaltet.

4. Betätigungseinheit nach Anspruch 1 oder 2, **dadurch gekennzeichnet,**
**dass** der Kraftsensor (28) ein Piezoelement ist.

5. Betätigungseinheit nach einem der Ansprüche 1 bis 4, **dadurch gekennzeichnet,**
**dass** der Kraftsensor (28) auf einer der Auflagefläche (20) gegenüberliegenden Innenfläche der Kapsel (12) angeordnet ist.

6. Betätigungseinheit nach einem der Ansprüche 1 bis 5, **dadurch gekennzeichnet,**
**dass** innerhalb der Kapsel (12) eine mit dem Kraftsensor (28) gekoppelte Signalaufbereitungsschaltung (30) aufgenommen ist.

7. Betätigungseinheit nach einem der Ansprüche 1 bis 6, **dadurch gekennzeichnet,**
**dass** die Kapsel (12) eine zylindrische Gestalt aufweist.

8. Betätigungseinheit nach einem der Ansprüche 1 bis 7, **dadurch gekennzeichnet,**
**dass** die Kapsel (12) eine topfförmige metallische Kappe (14) besitzt und dass ein die Kappe (14) abschließender Kappenboden (22) vorhanden ist, wobei die Auflagefläche (20) auf einer Oberseite der Kappe (14) ausgebildet ist.

9. Betätigungseinheit nach einem der Ansprüche 1 bis 8, **dadurch gekennzeichnet,**
**dass** die Kapsel (12) wenigstens eine Öffnung (26) aufweist, durch welche sich der elektrische Kontakt (24) hindurch erstreckt, wobei die Öffnung (26) mittels Glas hermetisch verschlossen ist.

10. Chirurgisches Elektrowerkzeug (50), umfassend ein Gehäuse (52), einen Elektromotor (60) sowie wenigstens eine fingerkraftgesteuerte und im Bereich des Gehäuses (52) angeordnete Betätigungseinheit (10), die eine hermetisch abgedichtete metallische Kapsel (12) zur Anordnung im Bereich des Gehäuses (52) des chirurgischen Elektrowerkzeugs (50), einen innerhalb der Kapsel (12) angeordneten Kraftsensor (28) und wenigstens einen aus der Kapsel (12) heraus führenden elektrischen Kontakt (24) umfasst, wobei die Kapsel (12) auf einer Aussenseite eine Auflagefläche (20) für einen Finger definiert und der Kraftsensor (28) sich in kraftübertragender Verbindung mit der Auflagefläche (20) befindet.

11. Chirurgisches Elektrowerkzeug nach Anspruch 10, **dadurch gekennzeichnet, dass** der Gehäuseeinsatz (12) eine hermetisch abgedichtete Kapsel (12) umfasst, innerhalb welcher der Kraftsensor (28) angeordnet ist, wobei die Auflagefläche (20) zur Krafteinleitung von einer Außenseite der Kapsel (12) gebildet ist.

12. Chirurgisches Elektrowerkzeug nach Anspruch 10 oder 11, **dadurch gekennzeichnet,**
**dass** eine erste Betätigungseinheit (10) zur Steuerung des Elektromotors (60) in einer ersten Drehrichtung und eine zweite Betätigungseinheit (10') zur Steuerung des Elektromotors (60) in einer zur ersten Drehrichtung entgegengesetzten zweiten Drehrichtung vorgesehen sind.

13. Chirurgisches Elektrowerkzeug nach einem der Ansprüche 10 bis 12, **dadurch gekennzeichnet,**
**dass** eine Signalaufbereitungsschaltung (30) mit einer Brückenschaltung (32) vorgesehen ist, wobei der Kraftsensor (28) einen Teil der Brückenschaltung (32) bildet.

14. Chirurgisches Elektrowerkzeug nach einem der Ansprüche 10 bis 13, **dadurch gekennzeichnet,**
**dass** das Elektrowerkzeug (50) ein langgestrecktes, zumindest annähernd zylindrisches Gehäuse (52) aufweist und die wenigstens eine Betätigungseinheit (10) in einem vorderen Gehäuseabschnitt angeordnet ist.

15. Verwendung eines metallischen Standard-Transistorgehäuses (12) oder von Teilen (22) hiervon zur hermetischen Verkapselung eines Kraftsensors (28) für eine Betätigungseinheit (10) eines chirurgischen Elektrowerkzeugs (50).

16. Verfahren zur Kalibrierung des Kraftsensors (28) der Betätigungseinheit (10) nach einem der Ansprüche 1 bis 9 und/oder des chirurgischen Elektrowerkzeugs (50) nach einem der Ansprüche 10 bis 14, umfassend die Schritte:
- Erfassen eines vorbestimmten Ereignisses;
- Messen eines Ausgangsignals des Kraftsensors (28) als Antwort auf das Erfassen des vorbestimmten Ereignisses; und
- Verwenden des gemessenen Ausgangssignals als Nullsignal für den nächsten Betätigungsvorgang.

17. Verfahren nach Anspruch 16, **dadurch gekennzeichnet, dass** das vorbestimmte Ereignis ein Aktivieren einer Spannungsversorgung ist.

18. Verfahren nach Anspruch 16 oder 17, **dadurch gekennzeichnet,**
**dass** das vorbestimmte Ereignis der Ablauf eines vorbestimmten Zeitintervalls ist, während dessen kein Betätigungsvorgang stattgefunden hat.

## Claims

1. Operating unit (10) for a surgical power tool (50), comprising:
- a hermetically sealed metal capsule (12) for arrangement in the region of a housing (52) of the surgical power tool (50), the capsule (12) defining on an outer face a contact surface (20) for a finger;
- a force sensor (28) which is arranged inside the capsule (12) and is in force-transmitting connection with the contact surface (20); and
- at least one electrical contact (24) leading out of the capsule (12).

2. Operating unit according to claim 1, **characterised in that** the contact surface (20) has a three-dimensional boundary which can be detected haptically.

3. Operating unit according to claim 1 or 2, **characterised in that** the force sensor (28) includes a strain gauge (97).

4. Operating unit according to claim 1 or 2, **characterised in that** the force sensor (28) is a piezo element.

5. Operating unit according to any one of claims 1 to 4, **characterised in that** the force sensor (28) is arranged on an inner face of the capsule (12) located opposite the contact surface (20).

6. Operating unit according to any one of claims 1 to 5, **characterised in that** a signal processing circuit (30) coupled to the force sensor (28) is housed inside the capsule (12).

7. Operating unit according to any one of claims 1 to 6, **characterised in that** the capsule (12) has a cylindrical shape.

8. Operating unit according to any one of claims 1 to 7, **characterised in that** the capsule (12) has a pot-shaped metal cap (14) and **in that** a cap base (22) closing the cap (14) is provided, the contact surface (20) being formed on an upper face of the cap (14).

9. Operating unit according to any one of claims 1 to 8, **characterised in that** the capsule (12) has at least one opening (26) through which the electrical contact (24) extends, the opening (26) being hermetically closed by means of glass.

10. Surgical power tool (50), comprising a housing (52), an electric motor (60) and at least one finger-force-controlled operating unit (10) which is arranged in the region of the housing (52) and comprises a hermetically sealed capsule (12) for arrangement in the region of a housing (52) of the surgical power tool (50), a force sensor (28) arranged inside the capsule (12) and at least one electrical contact (24) leading out of the capsule (12), the capsule (12) defining on an outer face a contact surface (20) for a finger, and the force sensor (28) being in force-transmitting connection with the contact surface (20).

11. Surgical power tool according to claim 10, **characterised in that** the housing insert (12) includes an hermetically sealed capsule (12) inside which the force sensor (28) is arranged, the contact surface (20) being configured for the transmission of force from an outer face of the capsule (12).

12. Surgical power tool according to claim 10 or 11, **characterised in that** a first operating unit (10) for controlling the electric motor (60) in a first direction of rotation, and a second operating unit (10') for controlling the electric motor (60) in a second direction of rotation opposite to the first direction of rotation, are provided.

13. Surgical power tool according to any one of claims 10 to 12, **characterised in that** a signal processing circuit (30) having a bridge circuit (32) is provided, the force sensor (28) forming part of the bridge circuit (32).

14. Surgical power tool according to any one of claims 10 to 13, **characterised in that** the power tool (50) has an elongated, at least approximately cylindrical housing (52) and the at least one operating unit (10) is arranged in a front housing section.

15. Use of a metal standard transistor housing (12) or of parts (22) thereof for hermetically encapsulating a force sensor (28) for an operating unit (10) of a surgical power tool (50).

16. Method for calibrating the force sensor (28) of the operating unit (10) according to any one of claims 1 to 9 and/or the surgical power tool (50) according to any one of claims 10 to 14, comprising the steps of:
- detecting a predetermined event;
- measuring an output signal of a force sensor (28) as a response to the detection of the predetermined event; and
- utilizing the measured output signal as the zero signal for the next operating process.

17. Method according to claim 16, **characterised in that** the predetermined event is an activation of a voltage supply.

18. Method according to claim 16 or 17, **characterised in that** the predetermined event is the expiry of a predetermined time interval during which no operating process has taken place.

## Revendications

1. Unité d'actionnement (10) pour un outil électrique chirurgical (50), comprenant :
- une capsule (12) métallique hermétiquement étanche pour l'agencement dans la région d'un boîtier (52) de l'outil électrique chirurgical (50), la capsule (12) définissant sur un côté extérieur une surface d'appui (20) pour un doigt ;
- un capteur de force (28) disposé à l'intérieur de la capsule (12), qui se trouve en liaison de transmission de force avec la surface d'appui (20) ; et
- au moins un contact (24) électrique sortant de la capsule (12).

2. Unité d'actionnement selon la revendication 1,
**caractérisée en ce que** la surface d'appui (20) présente une délimitation dans l'espace, qui peut être saisie par contact tactile.

3. Unité d'actionnement selon la revendication 1 ou 2,
**caractérisée en ce que** le capteur de force (28) contient une jauge d'extensométrie (97).

4. Unité d'actionnement selon la revendication 1 ou 2,
**caractérisée en ce que** le capteur de force (28) est un élément piézoélectrique.

5. Unité d'actionnement selon l'une quelconque des revendications 1 à 4,
**caractérisée en ce que** le capteur de force (28) est disposé sur une surface intérieure de la capsule (12), en face de la surface d'appui (20).

6. Unité d'actionnement selon l'une quelconque des revendications 1 à 5,
**caractérisée en ce que** un circuit de remise en forme de signal (30) couplé avec le capteur de force (28) est logé à l'intérieur de la capsule (12).

7. Unité d'actionnement selon l'une quelconque des revendications 1 à 6,
**caractérisée en ce que** la capsule (12) présente une forme cylindrique.

8. Unité d'actionnement selon l'une quelconque des revendications 1 à 7,
**caractérisée en ce que** la capsule (12) présente un capot (14) métallique en forme de pot, et **en ce qu'**un fond de capot (22) fermant le capot (14) est présent, la surface d'appui (20) étant ménagée sur un côté supérieur du capot (14).

9. Unité d'actionnement selon l'une quelconque des revendications 1 à 8,
**caractérisée en ce que** la capsule (12) présente au moins une ouverture (26) à travers laquelle s'étend le contact (24) électrique, l'ouverture (26) étant fermée de façon hermétique au moyen de verre.

10. Outil électrique chirurgical (50) comprenant un boîtier (52), un moteur électrique (60) et au moins une unité d'actionnement (10) commandée par une force de doigt et disposée dans la région du boîtier (52), qui comporte une capsule (12) métallique hermétiquement étanche pour l'agencement dans la région du boîtier (52) de l'outil électrique chirurgical (50), un capteur de force (28) disposé à l'intérieur de la capsule (12) et au moins un contact (24) électrique sortant de la capsule (12), la capsule (12) définissant sur un côté extérieur une surface d'appui (20) pour un doigt et le capteur de force (28) se trouvant en liaison de transmission de force avec la surface d'appui (20).

11. Outil électrique chirurgical selon la revendication 10,
**caractérisé en ce que** l'insert de boîtier (12) comporte une capsule (12) hermétiquement étanche, à l'intérieur de laquelle le capteur de force (28) est disposé, la surface d'appui (20) étant formée pour l'application de force par un côté extérieur de la capsule (12).

12. Outil électrique chirurgical selon la revendication 10 ou 11,
**caractérisé en ce qu'**une première unité d'actionnement (10) et une seconde unité d'actionnement (10') sont prévues respectivement pour la commande du moteur électrique (60) dans un premier sens de rotation et pour la commande du moteur électrique (60) dans un second sens de rotation opposé au premier sens de rotation.

13. Outil électrique chirurgical selon l'une quelconque des revendications 10 à 12,
**caractérisé en ce qu'**un circuit de remise en forme de signal (30) est prévu avec un circuit en pont (32), le capteur de force (28) formant une partie du circuit en pont (32).

14. Outil électrique chirurgical selon l'une quelconque des revendications 10 à 13,
**caractérisé en ce que** l'outil électrique (50) présente un boîtier (52) allongé, au moins approximativement cylindrique, et **en ce que** la au moins une unité d'actionnement (10) est disposée dans une partie de boîtier avant.

15. Utilisation d'un boîtier de transistor standard (12) métallique ou de parties (22) de ce boîtier pour l'encapsulage hermétique d'un capteur de force (28) pour une unité d'actionnement (10) d'un outil électrique chirurgical (50).

16. Procédé pour l'étalonnage du capteur de force (28) de l'unité d'actionnement (10) selon l'une quelconque des revendications 1 à 9 et/ou de l'outil électrique chirurgical (50) selon l'une quelconque des revendications 10 à 14, comprenant les étapes suivantes :
- enregistrement d'un événement prédéfini ;
- mesure d'un signal de sortie du capteur de force (28) comme réponse à l'enregistrement de l'événement prédéfini ; et
- utilisation du signal de sortie mesuré comme signal de sortie zéro pour la prochaine opération d'actionnement

17. Procédé selon la revendication 16,
**caractérisé en ce que** l'événement prédéfini est une activation d'une alimentation en tension.

18. Procédé selon la revendication 16 ou 17,
**caractérisé en ce que** l'événement prédéfini est l'écoulement d'un laps de temps prédéfini, pendant lequel aucune opération d'actionnement n'a eu lieu.
